Europäisches Patentamt

European Patent Office

Office européen des brevets

(19)

(11) Veröffentlichungsnummer: **0 337 256 B1**

(12) **EUROPÄISCHE PATENTSCHRIFT**

(45) Veröffentlichungstag der Patentschrift: **19.11.92**

(51) Int. Cl.⁵: **A61K 9/20**, A61J 3/10

(21) Anmeldenummer: **89105917.2**

(22) Anmeldetag: **05.04.89**

(54) **Verfahren zur Herstellung pharmazeutischer Mischungen.**

(30) Priorität: **15.04.88 DE 3812567**

(43) Veröffentlichungstag der Anmeldung:
**18.10.89 Patentblatt 89/42**

(45) Bekanntmachung des Hinweises auf die
Patenterteilung:
**19.11.92 Patentblatt 92/47**

(84) Benannte Vertragsstaaten:
**AT BE CH DE ES FR GB GR IT LI NL SE**

(56) Entgegenhaltungen:
**EP-A- 0 240 904**
**EP-A- 0 240 906**
**EP-A- 0 275 834**

(73) Patentinhaber: **BASF Aktiengesellschaft**
**Carl-Bosch-Strasse 38**
**W-6700 Ludwigshafen(DE)**

(72) Erfinder: **Klimesch, Roger, Dr.**
**Georg-Froeba-Strasse 43**
**W-6146 Alsbach-Haehnlein 2(DE)**
Erfinder: **Bleckmann, Gerhard**
**Giselherstrasse 9**
**W-6840 Lampertheim 5(DE)**
Erfinder: **Schlemmer, Lothar**
**Duisbergstrasse 1A**
**W-6701 Maxdorf(DE)**

EP 0 337 256 B1

## Beschreibung

Die Erfindung betrifft ein Verfahren zur Herstellung pharmazeutischer Mischungen durch kontinuierliches Einwiegen der Einzelkomponenten.

In der Pharmaindustrie werden bisher die einzelnen Komponenten diskontinuierlich abgewogen und gemischt. Wegen der Gefahr einer Entmischung im Falle von unterschiedlichen Schüttgewichten und/oder Fließeigenschaften müssen die Mischpartien relativ klein gehalten und Umfüllvorgänge und größere Transportwege vermieden werden. Dieses Verfahren ist umständlich und unwirtschaftlich, außerdem paßt es nicht zu modernen, kontinuierlichen Tablettierverfahren, die ohne eine (bisher stets diskontinuierliche) Granulierung auskommen. Solche Verfahren sind beispielsweise das Direkttablettieren von Wirkstoff/Hilfsstoffmischungen oder das Extrudieren von pharmazeutischen Mischungen, z.B. gemäß den deutschen Anmeldungen P 3612212.2 und 3612211.4.

Es besteht in der Pharmaindustrie ein Vorurteil gegen das kontinuierliche Dosieren von Komponenten zur Herstellung einer Mischung. In der Vergangenheit war dieses Vorurteil berechtigt, da die erforderliche Dosiergenauigkeit (±5 % vom Sollwert) nur über größere Zeiträume (mehrere Minuten), nicht aber innerhalb von Kurzzeiträumen (weniger als 1 Minute) gegeben war. Um auf den üblichen Tablettiermaschinen Tabletten herstellen zu können, deren Gleichmäßigkeit der Zusammensetzung den Vorschriften der Arzneibücher entspricht, ist eine hohe Konstanz der Dosierung jeder einzelnen Komponente im Sekundenbereich erforderlich.

Durch die Weiterentwicklung der Dosierwaagen unter Einsatz moderner Elektronik (neuester Mikroprozessortechnik) ist die zeitliche Konstanz der Dosierungen entscheidend verbessert worden. Es wurde nun gefunden, daß sie jetzt auch zum Einsatz in der Pharmaindustrie geeignet sind. Dies gilt insbesondere für den Einsatz bei modernen, kontinuierlichen Tablettierverfahren, wie das obengenante Extrusionsverfahren. Im Extruder oder auch im Schneckenteil einer Spritzgußmaschine findet eine weitere Verbesserung der Durchmischung statt, so daß gegebenenfalls auftretende Kurzzeitschwankungen in der Mischungszusamensetzung ausgeglichen werden. Deshalb werden diese Tablettierverfahren im Zusammenhang mit dem erfindungsgemäßen Dosierverfahren besonders bevorzugt.

Überraschenderweise wurde festgestellt, daß das erfindungsgemäße kontinuierliche Dosieren nicht nur entgegen dem in der Pharmaindustrie bestehenden Vorurteil praktikabel ist, sondern daß es in vielen Fällen sogar zu einer gleichmäßigeren Dosierung führt als nach dem herkömmlichen diskontinuierlichen Verfahren, weil hier im Gegensatz zu dort jede Möglichkeit einer Entmischung ausgeschlossen ist, denn die kontinuierliche Dosierung erfolgt unmittelbar am Ort der Tablettierung.

Über diesen entscheidenden Vorteil hinaus bringt das neue Verfahren erhebliche Vorteile hinsichtlich des Bedarfs an Raum, Zeit und Personal und ist somit bedeutend wirtschaftlicher.

Die kontinuierliche Dosierung von Komponenten ist zwar in der Kunststoffindustrie seit langem üblich, die Übertragung auf die Pharmaindustrie lag jedoch nicht nahe, da zwischen beiden Industrien normalerweise keinerlei Kontakt besteht und die Pharmaindustrie aufgrund ihrer hohen Anforderungen an die Dosiergenauigkeit derartige Verfahren für ihre Zwecke für ungeeignet hält.

Für die Beispiele wurde pro Komponente eine Differential-Dosierwaage der Fa. K-TRON Soder AG, Niederlenz, CH-5702, verwendet. Die einzelnen Komponenten wurden mit Hilfe dieser Waagen direkt in den Trichter eines Extruders, meist vom Typ ZSK 30 der Fa. Werner & Pfleiderer, Stuttgart-Feuerbach, oder einer Spritzgußmaschine dosiert. Der Durchsatz an Gesamtmischung lag stets im Bereich von 2 bis 5 kg/Stunde. Zur Kontrolle der Gleichmäßigkeit der Zusammensetzung der Tabletten wurden jeweils drei Tabletten auf ihren Gehalt an Wirkstoff und Hilfsstoffen analysiert. Auf eine Analyse des Gehalts an Polymeren wurde verzichtet, da dieser sich rechnerisch ergibt.

Die in den Beispielen genannten Teile und Prozente beziehen sich auf das Gewicht.

### Beispiel 1

45 Teile eines Copolymerisates vom K-Wert (nach H. Fikentscher, Cellulose-Chemie 13 (1932), S. 58-64 und 71-74) 30 aus 60 % N-Vinylpyrrolid-2-on (NVP) und 40 % Vinylacetat (Vac), 5 Teile Stearylalkohol und 50 Teile Theophyllin wurden über drei der oben genannten Dosierwaagen in den Trichter eines Extruders des oben genannten Typs dosiert und extrudiert. Die Temperatur des aus sechs Schüssen bestehenden Extrudermantels betrug 30, 60, 60, 60, 60 und 60°C; die Düse wurde auf 100°C aufgeheizt. Der hierbei erhaltene Strang wurde direkt mit dem in den Ansprüchen 5 und 6 der EP-A-240 906 und der zugehörigen Zeichnung beschriebenen Gerät zu Oblong-Tabletten verpreßt. Die Analyse dieser Tabletten hatte folgendes Ergebnis:

| Wirkstoff: | 49,9 | 50,2 | 49,7 % |
|---|---|---|---|
| Stearylalkohol: | 5,10 | 4,92 | 5,03 % |

Beispiel 2

50 Teile des Copolymerisats von Beispiel 1 und 50 Teile Theophyllin wurden gemäß Beispiel 1 in einem Doppelschneckenextruder vermischt und extrudiert und anschließend gemäß Beispiel 1 zu Oblong-Tabletten verpreßt. Die Temperatur der Extruderschüsse wurde auf 30, 60, 60, 60, 90 und 120°C eingestellt. Die Düse hatte ebenfalls eine Temperatur von 120°C.
Analyse des Wirkstoffgehaltes: 49,3, 50,1 und 50,5 %

Beispiel 3

47,5 Teile eines Copolymerisats vom K-Wert 30 aus 60 % NVP und 40 % Vac, 2,5 Teile vernetztes Polyvinylpyrrolidon (PVP) als Tablettensprengmittel und 50 Teile Theophyllin wurden gemäß Beispiel 1 in einem Doppelschneckenextruder vermischt und extrudiert und anschließend wie dort verformt. Die Temperatur der fünf Schüsse betrug jeweils 120°C, die Düse hatte eine Temperatur von 130°C.
Analyse des Wirkstoffgehaltes: 50,6, 50,1 und 49,8 %

Beispiel 4

50 Teile des Copolymerisates vom K-Wert 52 aus 30 % NVP und 70 % Vac und 50 Teile Theophyillin wurde gemäß Beispiel 1 im Doppelschneckenextruder gemischt und extrudiert und zu Tabletten verformt. Die Temperatur der fünf Schüsse betrug 30, 60, 100, 100 und 120°C. Die Düse wurde ebenfalls auf 120°C aufgeheizt.
Analyse des Wirkstoffgehalts: 50,8, 49,9 und 49,6 %.

Beispiele 5 bis 8

Eine Mischung von 50 % eines NVP-Homopolymeren von dem jeweils in der Tabelle angegebenen K-Wert und 50 % Theophyllin wurde gemäß Beispiel 1 in einen Einwellen-Extruder dosiert, bei der jeweils in der Tabelle angegebenen Temperatur aufgeschmolzen, extrudiert und gemäß Beispiel 1 zu Tabletten verformt. Sodann wurde der Wirkstoffgehalt von je drei Tabletten analysiert.

| Bei-spiel | K-Wert | T [°C] | | | | | | Theophyllingehalt (%) | | |
|---|---|---|---|---|---|---|---|---|---|---|
| | | 1. | 2. | 3. | 4. | 5. | Düse | | | |
| | | | | Schuß | | | | | | |
| 5 | 12 | 115 | 125 | 135 | 135 | 135 | 145 | 51,0 | 49,4 | 49,6 |
| 6 | 17 | 125 | 125 | 135 | 145 | 145 | 155 | 49,9 | 50,8 | 49,2 |
| 7 | 25 | 145 | 155 | 165 | 175 | 175 | 175 | 50,3 | 49,1 | 50,7 |
| 8 | 30 | 150 | 160 | 160 | 170 | 180 | 180 | 49,1 | 50,5 | 50,8 |

Beispiel 9

40 Teile eines Copolymerisates aus 60 % NVP und 40 % Vac vom K-Wert 30, 10 Teile Polyhydroxyethylmethacrylat und 50 Teile Theophyllin wurden gemäß Beispiel 1 verarbeitet. Temperatur der Schüsse: 70, 80, 80, 80, 80°C. Düse: 90°C. Wirkstoffgehalt: 50,2, 50,4 und 49,8 %.

Beispie 10

50 Teile eines handelsüblichen, zu 80 % verseiften Polyvinylacetats und 50 Teile Theophyllin wurden analog zu Beispiel 1 verarbeitet. Die Temperatur der Schüsse betrug 100, 100, 110, 120, 130°C. Düse: 150°C. Theophyllingehalt: 49,1, 50,9 und 49,8 %.

Beispie 11

50 Teile Polyhydroxyethylmethacrylat vom K-Wert 30 und 50 Teile Theophyllin wurden gemäß Beispiel 1 verarbeitet. Temperatur der Schüsse: 120, 130, 150, 160, 160°C. Düse: 170°C. Theophyllingehalt: 49,8, 50,4 und 50,1 %.

Beispie 12 bis 14

36 Teile eines Copolymerisates aus 60 % NVP und 40 % Vac vom K-Wert 30, 4 Teile Stearylalkohol, 40 Teile Theophyllin und 20 Teile
Beispiel 12) Stärke
Beispiel 13) Lactose
Beispiel 14) Saccharose
wurden gemäß Beispiel 1 in einen 6-schüssigen Doppelschneckenextruder dosiert, extrudiert und wie dort zu Tabletten verformt. Die Temperatur der Schüsse betrug 90, 100, 110, 120, 120, 130°C, die der Düse 135°C. Der Theophyllingehalt betrug
Beispiel 12) 50,0, 50,3, 50,1 %
Beispiel 13) 50,4, 49,9, 49,6 %
Beispiel 14) 49,9, 50,3, 49,7 %.

Beispiele 15 bis 17

50 Teile des Copolymerisates der Beispiele 12 bis 14 und 50 Teile Verapamil wurden gemäß den Beispielen 12 bis 14 zu Tabletten geformt. Verapamilgehalt:
Beispiel 15) 49,8, 49,6, 50,0 %
Beispiel 16) 50,1, 49,8, 50,4 %
Beispiel 17) 50,3, 50,5, 49,9 %.
Analog den obigen Beispielen wurden die folgenden durchgeführt. Die Bedingungen der Verarbeitung sowie die gefundenen Gehalte an Wirkstoff und monomerem Hilfsstoff sind tabellarisch erfaßt.

4

Tabelle

| Beisp. Nr. | Wirkstoff | Polymer | Hilfs-stoff | Gew.-Verhältnis Wirkst./Polymer/ Hilfsstoff | T1 | T2 | T3 | T4 | T5 | T6 | Düse |
|---|---|---|---|---|---|---|---|---|---|---|---|
| 18 | Pseudoephedrin 47,5 Diphenhydramin 2,5 | A | ./. | 50/50/0 | 60 | 80 | 100 | 120 | 120 | 120 | 120 |
| 19 | Propafenon | A | Stärke | 40/40/20 | 60 | 70 | 90 | 110 | 110 | 110 | 110 |
| 20 | Propafenon | A | StA | 60/35/5 | 80 | 90 | 100 | 120 | 140 | 140 | 140 |
| 21 | Propafenon | A | StA | 60/30/10 | 80 | 90 | 100 | 120 | 130 | 130 | 140 |
| 22 | Propafenon | A | StS | 60/35/5 | 70 | 90 | 100 | 110 | 115 | 115 | 115 |
| 23 | Propafenon | B | StA | 50/40/10 | 65 | 80 | 95 | 110 | 110 | 110 | 110 |
| 24 | Propafenon | A | MgSt | 60/35/5 | 60 | 70 | 80 | 80 | 95 | 100 | 100 |
| 25 | Propafenon | A | MgSt | 50/40/10 | 60 | 70 | 80 | 80 | 95 | 100 | 100 |
| 26 | Anipamil | A | MgSt | 50/40/10 | 30 | 30 | 40 | 40 | 60 | 60 | 60 |
| 27 | Vitamin B1 | B | ./. | 50/50/0 | 40 | 40 | 50 | 60 | 80 | 80 | 80 |
| 28 | Nicotinsäure | A | ./. | 50/50/0 | 60 | 70 | 80 | 95 | 95 | 100 | 100 |
| 29 | Biperiden | A | StA | 50/45/5 | 80 | 90 | 100 | 120 | 120 | 130 | 135 |
| 30 | Biperiden | A | ./. | 50/50/0 | 80 | 90 | 110 | 120 | 140 | 140 | 140 |
| 31 | Canthaxantin | B | ./. | 50/50/0 | 30 | 30 | 40 | 40 | 60 | 60 | 60 |
| 32 | Canthaxantin | A | ./. | 50/50/0 | 40 | 40 | 55 | 60 | 60 | 80 | 80 |

EP 0 337 256 B1

Tabelle - (Fortsetzung)

| Beisp. Nr. | Wirkstoffgehalt (%) | | | Hilfsstoffgehalt (%) | | |
|---|---|---|---|---|---|---|
| 18 | 47,6 | 47,1 | 47,8 | | | |
| | 2,55 | 2,48 | 2,53 | | | |
| 19 | 40,1 | 39,6 | 40,0 | | | |
| 20 | 59,6 | 59,9 | 60,4 | 5,05 | 5,02 | 4,97 |
| 21 | 60,3 | 60,1 | 59,5 | 9,92 | 10,08 | 10,10 |
| 22 | 60,5 | 59,8 | 60,6 | 4,93 | 4,99 | 5,00 |
| 23 | 50,0 | 49,4 | 50,3 | 10,10 | 10,09 | 10,01 |
| 24 | 60,8 | 60,1 | 60,3 | 5,05 | 4,99 | 4,98 |
| 25 | 49,8 | 50,2 | 50,5 | 9,95 | 9,94 | 10,03 |
| 26 | 50,7 | 49,5 | 49,4 | 10,06 | 10,00 | 9,98 |
| 27 | 49,3 | 49,9 | 50,2 | | | |
| 28 | 50,4 | 50,2 | 50,8 | | | |
| 29 | 50,2 | 50,0 | 49,6 | 4,96 | 4,98 | 5,05 |
| 30 | 49,4 | 49,9 | 50,1 | | | |
| 31 | 50,6 | 49,6 | 49,6 | | | |
| 32 | 50,1 | 49,7 | 50,5 | | | |

EP 0 337 256 B1

Tabelle - (Fortsetzung)

| Beisp. Nr. | Wirkstoff | Polymer | Gew.-Ver. Wirkst./ Polymer | T1 | T2 | T3 | T4 | T5 | T6 | Düse | Wirkstoffgehalt (%) | | |
|---|---|---|---|---|---|---|---|---|---|---|---|---|---|
| 33 | Indomethacin | A | 25/75 | 50 | 60 | 70 | 80 | 80 | 80 | 80 | 24,8 | 24,9 | 24,8 |
| 34 | Indomethacin | B | 25/75 | 60 | 80 | 100 | 120 | 120 | 120 | 120 | 25,2 | 25,0 | 25,1 |
| 35 | Anipamil | A | 25/75 | 30 | 30 | 40 | 50 | 50 | 60 | 60 | 24,9 | 24,7 | 25,2 |
| 36 | Anipamil | B | 25/75 | 30 | 30 | 40 | 50 | 50 | 60 | 60 | 25,0 | 25,2 | 24,9 |
| 37 | Benzocain | D | 25/75 | 60 | 80 | 95 | 100 | 120 | 120 | 140 | 25,3 | 24,8 | 25,0 |
| 38 | Benzocain | D | 25/75 | 60 | 80 | 95 | 100 | 120 | 130 | 140 | 24,8 | 25,1 | 24,8 |
| 39 | Benzocain | F | 25/75 | 30 | 30 | 40 | 50 | 50 | 60 | 60 | 24,9 | 24,9 | 24,9 |
| 40 | Benzocain | B | 25/75 | 60 | 80 | 100 | 120 | 120 | 120 | 120 | 24,8 | 25,0 | 25,2 |
| 41 | 5,5-Diphenhydramin | B | 25/75 | 60 | 80 | 100 | 120 | 120 | 120 | 120 | 25,0 | 25,3 | 25,0 |
| 42 | Paracetamid | B | 25/75 | 60 | 80 | 100 | 120 | 120 | 120 | 120 | 25,1 | 25,1 | 24,8 |
| 43 | Sulfathiazol | B | 25/75 | 70 | 90 | 100 | 100 | 100 | 100 | 120 | 24,8 | 24,7 | 24,8 |
| 44 | Sulfathiazol | E | 25/75 | 70 | 90 | 100 | 100 | 100 | 110 | 120 | 25,0 | 25,0 | 25,1 |
| 45 | Benzocain | A | 25/75 | 30 | 30 | 40 | 50 | 60 | 70 | 70 | 29,8 | 25,2 | 25,1 |
| 46 | 5,5-Diphenhydramin | A | 25/75 | 60 | 80 | 100 | 120 | 120 | 120 | 130 | 24,9 | 24,8 | 25,2 |
| 47 | Paracetamol | A | 25/75 | 60 | 80 | 100 | 120 | 120 | 120 | 130 | 25,0 | 24,9 | 24,8 |
| 48 | Sulfathiazol | A | 50/50 | 70 | 90 | 100 | 100 | 100 | 100 | 130 | 50,5 | 50,1 | 49,7 |
| 49 | Vitamin C | C | 50/50 | 75 | 95 | 95 | 120 | 120 | 120 | 120 | 49,3 | 49,8 | 50,1 |

EP 0 337 256 B1

EP 0 337 256 B1

Tabelle - (Fortsetzung)

| Beisp. Nr. | Wirkstoff | Polymer | Gew.-Verhältnis Wirkst./Polymer | T1 | T2 | T3 | T4 | T5 | T6 | Düse | Wirkstoffgehalt (%) | | |
|---|---|---|---|---|---|---|---|---|---|---|---|---|---|
| 50 | Benzocain | E | 25/75 | 60 | 70 | 80 | 120 | 130 | 130 | 130 | 24,9 | 24,7 | 24,9 |
| 51 | Benzocain | G | 25/75 | 60 | 70 | 70 | 80 | 80 | 80 | 120 | 25,0 | 24,8 | 25,1 |
| 52 | Benzocain | H | 25/75 | 50 | 60 | 60 | 60 | 80 | 90 | 110 | 25,2 | 25,2 | 25,0 |
| 53 | Benzocain | I | 25/75 | 50 | 60 | 70 | 70 | 75 | 75 | 80 | 24,9 | 25,3 | 25,1 |

Tabelle (Fortsetzung)

| Beisp. Nr. | Wirkstoff | Polymer | Hilfs- stoff | Gew.-Verhältnis Wirkst./Polymer/ Hilfsstoff | T1 | T2 | T3 | T4 | T5 | T6 | Düse |
|---|---|---|---|---|---|---|---|---|---|---|---|
| 54 | Metoprolol | A | StA | 40/55/5 | 60 | 70 | 80 | 80 | 90 | 80 | 80 |
| 55 | Ranitidin | A | – | 46/54 | 60 | 70 | 80 | 80 | 90 | 90 | 80 |
| 56 | Diclophenac | A | StA | 40/55/5 | 65 | 70 | 80 | 90 | 90 | 90 | 90 |
| 57 | Furosemid | A | StA | 30/60/10 | 65 | 75 | 80 | 90 | 100 | 100 | 100 |
| 58 | Nifedipin | A | StA | 20/70/10 | 60 | 70 | 80 | 80 | 80 | 80 | 80 |
| 59 | Gallopamil | A | StA | 40/54/6 | 50 | 60 | 70 | 80 | 80 | 70 | 70 |
| 60 | Gallopamil | A | StA | 40/48/12 | 50 | 60 | 70 | 80 | 80 | 70 | 70 |
| 61 | Gallopamil | A | StA | 40/42/18 | 50 | 60 | 70 | 80 | 80 | 70 | 70 |
| 62 | Gallopamil | A | StS | 40/54/6 | 50 | 60 | 70 | 80 | 80 | 70 | 70 |
| 63 | Gallopamil | A | StS | 40/48/12 | 50 | 60 | 70 | 80 | 80 | 70 | 70 |
| 64 | Gallopamil | A | StS | 40/42/18 | 50 | 60 | 70 | 80 | 80 | 70 | 70 |
| 65 | Anipamil | A | StA | 34/54,4/13,6 | 50 | 60 | 65 | 65 | 60 | 60 | 55 |
| 66 | Biperiden | A | StA | 6/89/5 | 45 | 55 | 60 | 65 | 65 | 65 | 60 |
| 67 | Biperiden | A | StA | 6/84/10 | 45 | 55 | 50 | 65 | 65 | 65 | 60 |
| 68 | Biperiden | A | StA | 6/79/15 | 45 | 55 | 60 | 65 | 65 | 65 | 60 |
| 69 | Biperiden | A | StA | 6/74/20 | 50 | 50 | 60 | 60 | 50 | 50 | 50 |
| 70 | Biperiden | A | StA | 6/69/25 | 40 | 50 | 55 | 60 | 60 | 50 | 50 |
| 71 | Biperiden | A | StA | 6/64/30 | 40 | 50 | 55 | 60 | 60 | 50 | 50 |
| 72 | Biperiden | A | StA | 6/59/35 | 40 | 50 | 55 | 60 | 60 | 50 | 50 |
| 73 | Bezafibrat | A | – | 61,5/38,5 | 60 | 70 | 80 | 80 | 80 | 80 | 80 |
| 74 | Bezafibrat | A | StA | 61,5/34/4,5 | 60 | 70 | 80 | 80 | 80 | 70 | 70 |
| 75 | Bezafibrat | A | StA | 61,5/29,5/9,0 | 40 | 45 | 50 | 50 | 50 | 50 | 50 |
| 76 | Metoprolol | A | Stärke | 40/45/15 | 60 | 70 | 80 | 80 | 80 | 80 | 80 |
| 77 | Metoprolol | A | Stärke | 40/35/25 | 55 | 60 | 65 | 70 | 70 | 70 | 70 |

EP 0 337 256 B1

Tabelle - (Fortsetzung)

| Beisp. Nr. | Wirkstoffgehalt (%) | | | Hilfsstoffgehalt (%) | | |
|---|---|---|---|---|---|---|
| 54 | 39,7 | 39,8 | 40,1 | 4,99 | 5,00 | 5,08 |
| 55 | 46,2 | 46,5 | 46,0 | | | |
| 56 | 40,2 | 40,4 | 40,2 | 4,93 | 4,95 | 5,01 |
| 57 | 29,7 | 30,1 | 30,2 | 10,0 | 10,1 | 10,1 |
| 58 | 20,2 | 20,0 | 19,9 | 9,9 | 10,1 | 10,1 |
| 59 | 39,9 | 40,4 | 39,7 | 5,93 | 6,04 | 6,05 |
| 60 | 40,1 | 39,7 | 40,4 | 12,1 | 12,1 | 12,0 |
| 61 | 39,7 | 40,0 | 39,9 | 17,8 | 18,1 | 18,7 |
| 62 | 40,3 | 40,1 | 39,8 | 5,93 | 5,93 | 6,09 |
| 63 | 39,8 | 40,3 | 40,4 | 12,0 | 12,0 | 11,9 |
| 64 | 40,4 | 39,9 | 39,7 | 17,9 | 17,8 | 18,0 |
| 65 | 34,4 | 34,3 | 33,8 | 13,5 | 13,7 | 13,6 |
| 66 | 5,99 | 6,07 | 6,09 | 5,06 | 5,07 | 5,00 |
| 67 | 5,92 | 6,05 | 6,06 | 9,9 | 9,9 | 10,0 |
| 68 | 6,06 | 6,01 | 5,92 | 14,9 | 14,9 | 15,2 |
| 69 | 5,98 | 5,94 | 5,99 | 19,8 | 20,0 | 19,9 |
| 70 | 5,93 | 5,98 | 5,99 | 25,1 | 25,2 | 25,0 |
| 71 | 5,94 | 6,07 | 5,97 | 30,1 | 30,3 | 29,7 |
| 72 | 5,94 | 6,09 | 6,07 | 34,9 | 35,6 | 34,8 |
| 73 | 62,0 | 61,8 | 61,2 | | | |
| 74 | 62,1 | 61,9 | 60,9 | 4,45 | 4,47 | 4,55 |
| 75 | 61,4 | 60,9 | 61,9 | 8,93 | 9,04 | 8,98 |
| 76 | 40,4 | 39,8 | 40,0 | | | |
| 77 | 40,2 | 40,1 | 39,7 | | | |

EP 0 337 256 B1

Tabelle - Fortsetzung

| Beisp. Nr. | Wirkstoff | Polymer | Hilfs-stoff | Gew.-Verhältnis Wirkst./Polymer/Hilfsstoff | T1 | T2 | T3 | T4 | T5 | T6 | Düse |
|---|---|---|---|---|---|---|---|---|---|---|---|
| 78 | Anipamil | A | Lactose | 32/43/25 | 55 | 60 | 70 | 80 | 70 | 70 | 65 |
| 79 | Anipamil | A | Cellulose | 32/61,2/6,8 | 55 | 60 | 70 | 80 | 65 | 65 | 60 |
| 80 | Anipamil | A | Lactose | 32/34,4/13,6 | 55 | 60 | 70 | 80 | 65 | 65 | 60 |
| 81 | Anipamil | A | Stärke | 32/54,4/13,6 | 55 | 60 | 70 | 80 | 65 | 65 | 60 |
| 82 | Coffein Pulver | A | StA | 50/45/5 | 65 | 75 | 90 | 90 | 90 | 90 | 100 |
| 83 | Coffein Pulver | A | - | 50/50 | 65 | 75 | 90 | 90 | 90 | 90 | 100 |
| 84 | Coffein Granulat | A | StA | 50/45/5 | 65 | 70 | 70 | 75 | 75 | 90 | 80 |
| 85 | Coffein Granulat | A | - | 50/50 | 65 | 70 | 70 | 75 | 75 | 90 | 80 |

| | | |
|---|---|---|
| A | = | Copolymer aus 60 Gew.% NVP und 40 Gew.% Vinylacetat, K-Wert ca. 33 |
| B | = | PVP, K-Wert 12 |
| C | = | PVP, K-Wert 17 |
| D | = | Mowiol® 30-92 (zu 92 % hydrolys. Polyvinylalkohol) |
| E | = | Mowiol 4-80 (zu 80 % hydrolys. Polyvinylalkohol) |
| F | = | Copolymer aus NVP, Vinylacetat und Hydroxypropylacrylat im Gewichtsverhältnis 30/40/30; K-Wert ca. 18 |
| G | = | Celluloseacetat |
| H | = | Celluloseacetat-phthalat |
| I | = | Copolymer Vinylacetat/Crotonsäure; K-Wert ca. 30 |
| StA | = | Stearylalkohol |
| StS | = | Stearinsäure |
| MgSt | = | Magnesiumstearat |

EP 0 337 256 B1

Tabelle – Fortsetzung

| Beisp. Nr. | Wirkstoffgehalt (%) | | | Hilfsstoffgehalt (%) | | |
|---|---|---|---|---|---|---|
| 78 | 31,8 | 32,0 | 32,2 | | | |
| 79 | 32,0 | 31,9 | 32,2 | | | |
| 80 | 32,3 | 32,2 | 31,8 | | | |
| 81 | 32,0 | 31,8 | 32,0 | | | |
| 82 | 50,4 | 50,2 | 50,3 | 5,06 | 4,97 | 4,95 |
| 83 | 50,2 | 50,3 | 49,8 | | | |
| 84 | 50,0 | 49,5 | 49,6 | 4,95 | 4,98 | 5,03 |
| 85 | 50,2 | 50,5 | 49,8 | | | |

Beispiel 86

50 Teile eines Copolymerisates vom K-Wert 30 aus 60 % NVP und 40 % Vac und 50 Teile Theophyllin wurden kontinuierlich in den Trichter einer Spritzgußmaschine dosiert und bei 120 °C zu Oblong-Tabletten von 1 cm Länge verarbeitet. Wirkstoffgehalt: 50,8, 49,4 und 50,5 %.

Beispiel 87

47,5 Teile des Copolymerisates von Beispiel 86, 2,5 Teile Stearylalkohol und 50 Teile Theophyllin wurden kontinuierlich in eine Spritzgußmaschine dosiert und bei 100 °C zu Dragee-Kernen verarbeitet. Die Form wurde bei Raumtemperatur belassen. Theophyllingehalt: 49,0, 50,3 und 50,9 %. Gehalt an Stearylalkohol 2,56, 2,49 und 2,44 %.

Beispiel 88

25 % Paracetamol und 75 % PVP vom K-Wert 12, das gemäß DE-A-36 42 633 in Wasser mit einem organischen Peroxid als Starter hergestellt worden war, wurden kontinuierlich in den Einfüllstutzen einer Spritzgußmaschine dosiert und bei einer Düsentemperatur von 130°C zu Dragee-Kernen geformt. Wirkstoffgehalt: 24,7, 25,2 und 24,9 %.

Beispiel 89 und 90

Beispiel 88 wurde mit Phenytoin bzw. Benzocain als Wirkstoff wiederholt. Wirkstoffgehalte:
Beispiel 89) 25,1, 25,3 und 24,9 % Phenytoin
Beispiel 90) 24,8, 25,2 und 24,5 % Benzocain.

**Patentansprüche**

1. Verfahren zur Herstellung pharmazeutischer Mischungen durch kontinuierliches Dosieren der Komponenten und Formgeben nach üblichen Methoden.

2. Verfahren zur Herstellung pharmazeutischer Mischungen nach Anspruch 1 durch kontinuierliches Dosieren der Komponenten in einen Extruder und Verformen des extrudierten plastischen Stranges nach üblichen Methoden.

3. Verfahren zur Herstellung pharmazeutischer Mischungen nach Anspruch 1 durch kontinuierliches Dosieren der Komponenten in einen Zweiwellenextruder und Verformen des extrudierten plastischen Stranges nach üblichen Methoden.

4. Verfahren zur Herstellung pharmazeutischer Mischungen nach Anspruch 1 durch kontinuierliches Dosieren der Komponenten in den Trichter einer Spritzgußmaschine und Spritzgießen.

5. Verfahren zur Herstellung pharmazeutischer Mischungen nach einem der Ansprüche 1 bis 4 durch kontinuierliches Dosieren der Komponenten auf elektronischen Differentialdosierwaagen mit Schneckenförderer.

6. Verfahren zur Herstellung pharmazeutischer Mischungen nach Anspruch 5 durch kontinuierliches Dosieren der Komponenten auf elektronischen Differentialdosierwaagen mit kämmenden, selbstreinigenden Doppelschnecken als Förderelement.

7. Verfahren nach einem der Ansprüche 1 bis 6, wobei die pro Stunde zu dosierenden Einzelmengen bei 50 g oder mehr liegen.

**Claims**

1. A process for the preparation of a pharmaceutical mixture by continuous metering of the components and shaping by a conventional method.

2. A process for the preparation of a pharmaceutical mixture as claimed in claim 1 by continuous metering of the components into an extruder and molding of the extruded plastic extrudate by a conventional method.

3. A process for the preparation of a pharmaceutical mixture as claimed in claim 1 by continuous metering of the components into a twin-screw extruder and molding of the extruded plastic extrudate by a conventional method.

4. A process for the preparation of a pharmaceutical mixture as claimed in claim 1 by continuous metering of the components into the hopper of an injection molding machine and carrying out injection molding.

5. A process for the preparation of a pharmaceutical mixture as claimed in any of claims 1 to 4 by continuous metering of the components on electronic differential metering balances having screw conveyors.

6. A process for the preparation of a pharmaceutical mixture as claimed in claim 5 by continuous metering of the components on electronic differential metering balances having intermeshing, self-purging twin screws as a conveying element.

7. A process as claimed in any of claims 1 to 6, wherein the individual amounts to be metered per hour are 50 g or more.

**Revendications**

1. Procédé de préparation de mélanges pharmaceutiques par dosage des composants en continu et mise en forme selon des méthodes usuelles.

2. Procédé de préparation de mélanges pharmaceutiques selon la revendication 1, par dosage des composants en continu, dans une boudineuse, et mise en forme du jonc plastique, extrudé, selon des méthodes usuelles.

3. Procédé de préparation de mélanges pharmaceutiques selon la revendication 1, par dosage des composants en continu, dans une boudineuse à deux arbres, et mise en forme du jonc plastique, extrudé, selon des méthodes usuelles.

4. Procédé de préparation de mélanges pharmaceutiques selon la revendication 1, par dosage des composants en continu, dans la trémie d'une presse à injecter, et moulage par injection.

5. Procédé de préparation de mélanges pharmaceutiques selon l'une des revendications 1 à 4, par dosage des composants en continu, sur des balances à doser différentielles à propulseur à vis sans fin.

6. Procédé de préparation de mélanges pharmaceutiques selon la revendication 5, par dosage des composants en continu, sur des balances à doser différentielles à doubles vis sans fin peignantes, auto-nettoyantes, en tant qu'élément transporteur.

7. Procédé selon l'une des revendications 1 à 6, avec lequel les quantités individuelles à doser par heure s'élèvent à 50 g ou plus.